# EUROPÄISCHE PATENTSCHRIFT

(11) **EP 0 706 988 B1**
(45) Veröffentlichungstag und Bekanntmachung des Hinweises auf die Patenterteilung: **15.04.1998**
(21) Anmeldenummer: 95115847.6
(22) Anmeldetag: 09.10.1995
(51) Int. Cl.: C07C 67/08, C07C 69/24, C07C 69/52

(54) **Verfahren zum Erzeugen von Fettsäure-Methylester oder Fettsäure-Äthylester und Glycerin durch Umesterung von Öl oder Fett**
Process for the production of methyl esters of fatty acids or ethyl esters of fatty acids and glycerine by transesterification of oil or fat
Procédé de production d'esters méthyliques d'acides gras ou d'esters éthyliques d'acides gras et de glycérine par transestérification d'huile ou de graisse

(30) Priorität: 13.10.1994 DE 4436517
(43) Veröffentlichungstag der Anmeldung: 17.04.1996
(73) Patentinhaber: METALLGESELLSCHAFT AG, 60323 Frankfurt am Main (DE)
(72) Erfinder: Buchold, Henning, Dr., D-63452 Hanau (DE); König, Peter, Dr., D-60439 Frankfurt am Main (DE); Ludwig, Ernst, D-61440 Oberursel (DE); Kramer, Wolfgang, D-61118 Bad Vilbel (DE)

(56) Entgegenhaltungen:
- EP-A- 0 131 991
- EP-A- 0 523 767

## Beschreibung

Die Erfindung betrifft ein Verfahren zum Umsetzen von Öl oder Fett mit Methanol oder Äthanol und einem alkalischen Katalysator in flüssiger Phase zu den Produkten Fettsäure-Methylester oder Fettsäure-Äthylester und Glycerin, die getrennt gewonnen werden, in einer oder mehreren Umsetzungsstufen, wobei jede Umsetzungsstufe eine Mischzone und einen Abscheider zum Abtrennen einer leichten, esterreichen Phase und einer schweren, glycerinreichen Phase aufweist, wobei der Mischzone der ersten Umsetzungsstufe Öl oder Fett sowie Methanol oder Äthanol und alkalischer Katalysator zugeführt werden und aus der in der ersten Umsetzungsstufe gewonnenen glycerinreichen Phase Glycerin abgetrennt wird. Als Ausgangsmaterial wird zumeist ein pflanzliches Öl oder Fett verwendet werden, doch ist auch ein fließfähiges tierisches Öl oder Fett nicht ausgeschlossen.

Ein solches Verfahren ist aus EP-A-0 523 767 (dazu korrespondiert DE-A-4 123 928) bekannt. Als Katalysator verwendet man hierbei bevorzugt Natriummethylat, das aber relativ teuer ist. Wenn man statt dessen NaOH oder KOH als Katalysator in die Mischzonen der Umsetzungsstufen gibt, werden störende Mengen an Nebenprodukten, insbesondere Seifen, gebildet.

Der Erfindung liegt die Aufgabe zugrunde, beim eingangs genannten verfahren möglichst kostengünstig zu arbeiten und dabei einen Katalysator zu verwenden, der praktisch nicht zu Verlusten durch Verseifung führt. Gleichzeitig soll das aus EP-A-0 523 767 bekannte Verfahren weiterentwickelt werden.

Erfindungsgemäß wird die Aufgabe beim eingangs genannten Verfahren dadurch gelöst, daß man aus der Umsetzung gewonnenes Glycerin mit Alkalihydroxid mischt und eine alkalische, wasserhaltige Lösung erzeugt, die Glycerin und Alkalihydroxid im molaren Verhältnis von 1 : 5 bis 10 : 1 enthält, daß man Wasser aus der alkalischen Lösung entfernt, wobei Mono-Alkali-Glycerat gebildet wird, und die glycerathaltige Lösung in mindestens eine Mischzone leitet, wobei der gesamte Katalysatorbedarf der Umsetzung zu 50 bis 100 % gedeckt wird. Als Alkalihydroxid verwendet man üblicherweise NaOH oder KOH.

In großtechnischen Anlagen arbeitet man in den meisten Fällen mit zwei oder drei Umsetzungsstufen, wie das bereits auch aus EP-A-0 523 767 bekannt ist. Die Menge des insgesamt den verschiedenen Umsetzungsstufen zugeführten Methanols und Äthanols liegt im Bereich vom 1- bis 3-fachen der stöchiometrisch notwendigen Menge. Wenn man mit zwei Umsetzungsstufen arbeitet, ist es zweckmäßig, der Mischzone der ersten Umsetzungsstufe 20 bis 60 % des gesamten Methanols oder Äthanols zuzuführen. Den Rest des Methanols oder Äthanols gibt man in die Mischzone der zweiten Umsetzungsstufe.

Zur Herstellung der den Katalysator enthaltenden Lösung verwendet man aus der Umsetzung gewonnenes Glycerin und NaOH oder KOH, so daß dieser Katalysator ersichtlich auf sehr kostengünstige Weise erzeugt wird. Dieses im Verfahren anfallende Glycerin wird mit einer Menge an Alkalihydroxid gemischt, die üblicherweise 0,1 bis 1,0 Gew.-% der Menge des umzusetzenden Öls oder Fettes entspricht. Auf diese Weise kann mindestens 50 % und vorzugsweise mindestens 80 % des gesamten Katalysatorbedarfs der Umsetzung durch die im Verfahren erzeugte glycerathaltige Lösung gedeckt werden. Üblicherweise wird nur während der Anfahrphase des Verfahrens, wenn noch kein Glycerin produziert ist, mit fremderzeugtem Katalysator, z.B. Na-Methylat, gearbeitet werden müssen. Die voll in Betrieb befindliche Anlage ist dann in der Lage, unter Zugabe von NaOH oder KOH im Verfahren selbst die glycerathaltige Lösung zu erzeugen, die im wesentlichen aus Glycerin und Mono-Alkali-Glycerat besteht und den gesamten Katalysatorbedarf decken kann.

Zu jeder Umsetzungsstufe gehört eine Mischzone, wobei diese Mischzone z.B. durch einen Rührbehälter oder auch durch mehrere, aufeinanderfolgende Rührbehälter gebildet sein kann.

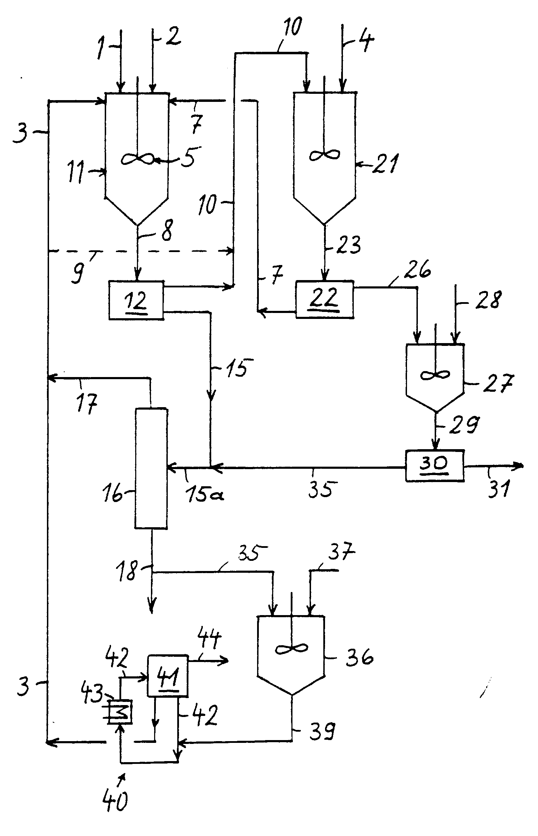

Ausgestaltungsmöglichkeiten des Verfahrens mit zwei Umsetzungsstufen werden mit Hilfe der Zeichnung erläutert. Hierbei wird Methanol als Alkohol verwendet, doch kann das Verfahren in gleicher Weise auch mit Äthanol zur Erzeugung von Äthylester durchgeführt werden.

Die erste Umsetzungsstufe weist den Mischreaktor (11) und den Schwerkraftabscheider (12) auf, zur zweiten Umsetzungsstufe gehören der Mischreaktor (21) und der Schwerkraftabscheider (22). Das zu behandelnde Öl oder Fett fließt durch die Leitung (1) in den Mischreaktor (11). Dem Reaktor (11) führt man ferner durch die Leitung (2) weitgehend wasserfreies Methanol sowie durch die Leitung (3) eine glycerathaltige Katalysator-Lösung zu. In der Leitung (7) wird schwere, glycerinhaltige Phase aus der zweiten Umsetzungsstufe in die erste Umsetzungsstufe zurückgeführt.

Als Schwerkraftabscheider kommen z.B. Faserbettabscheider infrage, die an sich bekannt und in "Filtration & Separation" (September/Oktober 1990), Seiten 360 bis 363, beschrieben sind.

Im Reaktor (11) erfolgt eine intensive Durchmischung mit Hilfe einer Rühreinrichtung (5). Das flüssige Produkt der Umsetzung im Reaktor (11) wird durch die Leitung (8) dem Abscheider (12) aufgegeben, aus dem man in der Leitung (10) eine leichte, esterreiche Phase abzieht. Getrennt davon wird in der Leitung (15) eine schwere, glycerinreiche Phase abgeleitet. Zusammen mit der esterreichen Phase der Leitung (10) gibt man dem Reaktor (21) durch die Leitung (4) Methanol auf. Einen Teil der Katalysatorlösung der Leitung (3) kann man bei Bedarf in der gestrichelt eingezeichneten Leitung (9) abzweigen und sie über die Leitung (10) in die zweite Umsetzungsstufe führen.

Die Temperaturen in den Reaktoren (11) und (21) sind ungefähr gleich, sie liegen im Reaktor (21) üblicherweise im Bereich von 50 bis 90°C und im Reaktor (21) im Bereich von 30 bis 80°C.

Das flüssige Produkt des Reaktors (21) wird in der Leitung (23) zum Abscheider (22) geführt, wobei die schwere, glycerinreiche Phase in der Leitung (7) abgezogen wird. Die leichte, esterreiche Phase gelangt in der Leitung (26) zu einem Extraktor (27), dem man durch die Leitung (28) Wasser zuführt.

Im Extraktor (27) mischt man Wasser mit der esterreichen Phase, dabei nimmt das Wasser die wasserlöslichen Substanzen, vor allem Methanol und Glycerin, auf und zerstört den Katalysator. Das im Extraktor (27) erzeugte Gemisch führt man durch die Leitung (29) zu einer Zentrifuge (30), aus der man durch die Leitung (31) Fettsäure-Methylester mit geringem Wassergehalt abzieht. In der Leitung (35) fällt eine wäßrige Phase an, die Methanol und Glycerin enthält und praktisch frei von Methylester ist. Diese wäßrige Phase gibt man zusammen mit der glycerinreichen Phase der Leitung (15) durch die Leitung (15a) einer Rektifikationskolonne (16) auf. Aus der Kolonne (16) gewinnt man über Kopf weitgehend wasserfreies Methanol, das man in der Leitung (17) abzieht und durch die Leitung (3) in den Reaktor (11) zurückführt.

Aus dem Sumpf der Kolonne (16) zieht man in der Leitung (18) rohes Glycerin ab. Einen Teilstrom dieses Glycerins gibt man durch die Leitung (35) einem Lösebehälter (36) auf, dem man durch die Leitung (37) auch NaOH (oder KOH) zuführt. Das Alkalihydroxid wird im Behälter (36) unter Rühren gelöst. Dabei wird ein Teil des Glycerins zu Mono-Natrium-Glycerat und Wasser umgesetzt. Abweichend von der Zeichnung kann man auch einen Teilstrom der glycerinreichen Phase der Leitung (15) in den Behälter (36) leiten.

In der Leitung (39) zieht man eine alkalische Lösung ab, die Glycerin und Na-Hydroxid im molaren Verhältnis von 1 : 5 bis 10 : 1 enthält. Durch das Entfernen von Wasser erzeugt man das Mono-Natrium-Glycerat, und zwar entsteht pro Mol Alkalihydroxid 1 Mol Mono-Natrium-Glycerat. In der Zeichnung ist hierfür ein Fallfilmverdampfer (40) vorgesehen, der einen Ausdampfbehälter (41), eine Kreislaufleitung (42), einen Erhitzer (43) und eine Abzugsleitung (44) für Wasserdampf besitzt. Weitgehend entwässerte Lösung zieht man in der Leitung (3) ab, der Wassergehalt dieser Lösung liegt bei höchstens 0,5 Gew.-% und vorzugsweise bei höchstens 0,1 Gew.-%. Da das Wasser zur Seifenbildung in den Reaktoren (11) und (21) führt, wird man Wert auf die Erzeugung einer möglichst wasserfreien Lösung in der Leitung (3) legen.

Die Entwässerung der alkalischen Lösung der Leitung (39) kann auch auf eine andere, an sich bekannte Weise erfolgen, z.B. durch Sprühtrocknung oder mit Hilfe eines Molekularsiebs.

### Beispiele

Im Labormaßstab wird in Analogie zur Zeichnung mit zwei Umsetzungsstufen gearbeitet, d.h. mit zwei Rührbehältern (11) und (21), in denen die Temperatur auf 60°C gehalten ist. Die Schwerkraftabscheider (12) und (22) sind Absetzbehälter, und an die Stelle der Kolonne (16) und des Fallfilmverdampfers (40) tritt jeweils ein Rotationsverdampfer. Auf die Leitungen (4), (9), (17) und (35) wird verzichtet. Man verwendet in allen Beispielen entsäuertes, entschleimtes und getrocknetes Rapsöl, das in einer Menge von 2,5 kg/h durch die Leitung (1) in den Rührbehälter (11) gegeben wird, zusammen mit 0,54 kg/h Methanol durch die Leitung (2).

### Beispiel 1

Im Rührbehälter (36) wird aus Glycerin und KOH im Gewichtsverhältnis von 4 : 1 eine Katalysatorlösung hergestellt, die man in einer Menge von 0,1 kg/h durch die Leitung (3) in den Rührbehälter (11) leitet und die ein molares Verhältnis Glycerin : Mono-Kalium-Glycerat von 2,4 : 1 sowie einen Wassergehalt von 0,2 Gew.-% aufweist. In der Leitung (29) fällt Methylester an, in welchem 5,5 Gew.-% Methanol, 0,8 Gew.-% freies Glycerin und 0,1 Gew.-% gebundenes Glycerin (Mono-, Di- und Triglyceride) gelöst sind. Die Glycerinphase in der Leitung (15) enthält 81,1 Gew.-% Glycerin, 12,7 Gew.-% Methanol, 4,4 Gew.-% K-Ionen, 0,1 Gew.-% Wasser und 1,7 Gew.-% Seifen.

### Beispiel 2 (Vergleichsbeispiel)

Beispiel 1 wird wiederholt, es wird jedoch auf die Katalysatorlösung der Leitung (3) verzichtet und stattdessen dem Rührbehälter (11) pro Stunde 20 g KOH, das in Methanol gelöst ist, zugeführt. In dem als Produkt erzeugten, in der Leitung (29) anfallenden Methylester sind 5,5 Gew.-% Methanol, 0,8 Gew.-% freies Glycerin und 0,1 Gew.-% gebundenes Glycerin gelöst. Die Glycerinphase der Leitung (15) enthält 60 Gew.-% Glycerin, 19,7 Gew.-% Methanol, 1,6 Gew.-% KOH, 1,0 Gew.-% Wasser und 17,7 Gew.-% Seifen. Die Seifenbildung entspricht einem Ölverlust von 2,6 Gew.-%.

### Beispiel 3 (Vergleichsbeispiel)

Die Arbeitsweise des Beispiels 2 wird so abgewandelt, daß man dem Rührbehälter (11) nunmehr als Katalysator pro Stunde 20 g KOH zuführt, das man im Methanol der Leitung (2) löst. Diese Lösung wird vor dem Einleiten in den Rührbehälter (11) zum Trocknen über ein Molekularsieb geleitet. Im Methylester der Leitung (29) sind nunmehr 7,5 Gew.-% Methanol, 0,5 Gew.-% freies Glycerin und 0,16 Gew.-% gebundenes Glycerin gelöst. Die Glycerinphase der Leitung (15) besteht aus 80,8 Gew.-% Glycerin, 11,9 Gew.-% Methanol, 4,1 Gew.-% K-Ionen, 0,2 Gew.-% Wasser und 3,0 Gew.-% Seifen.

## Patentansprüche

1. Verfahren zum Umsetzen von Öl oder Fett mit Methanol oder Äthanol und einem alkalischen Katalysator in flüssiger Phase zu den Produkten Fettsäure-Methylester oder Fettsäure-Äthylester und Glycerin, die getrennt gewonnen werden, in einer oder mehreren Umsetzungsstufen, wobei jede Umsetzungsstufe eine Mischzone und einen Abscheider zum Abtrennen einer leichten, esterreichen Phase und einer schweren, glycerinreichen Phase aufweist, wobei der Mischzone der ersten Umsetzungsstufe Öl oder Fett sowie Methanol oder Äthanol und alkalischer Katalysator zugeführt werden und aus der in der ersten Umsetzungsstufe gewonnenen glycerinreichen Phase Glycerin abgetrennt wird, dadurch gekennzeichnet, daß man aus der Umsetzung gewonnenes Glycerin mit Alkalihydroxid mischt und eine alkalische, wasserhaltige Lösung erzeugt, die Glycerin und Alkalihydroxid im molaren Verhältnis von 1 : 5 bis 10 : 1 enthält, daß man Wasser aus der alkalischen Lösung entfernt, wobei Mono-Alkali-Glycerat gebildet wird, und die glycerathaltige Lösung in mindestens eine Mischzone leitet, wobei der gesamte Katalysatorbedarf der Umsetzung zu 50 bis 100 % gedeckt wird.

2. Verfahren nach Anspruch 1, dadurch gekennzeichnet, daß man aus der Umsetzung gewonnenes Glycerin mit einer Menge an Alkalihydroxid mischt, die 0,1 bis 1,0 Gew.-% der Menge des umzusetzenden Öls oder Fettes entspricht.

3. Verfahren nach Anspruch 1 oder 2, dadurch gekennzeichnet, daß die glycerathaltige Lösung, die man in mindestens eine Mischzone leitet, höchstens 0,5 Gew.-% Wasser enthält.

4. Verfahren nach Anspruch 1 oder einem der folgenden, dadurch gekennzeichnet, daß der gesamte Katalysatorbedarf der Umsetzung des Öls oder Fettes zu mindestens 80 % durch die glycerathaltige Lösung gedeckt wird.

## Claims

1. A process for the conversion of oil or fat with methanol or ethanol and an alkaline catalyst in the liquid phase to the products fatty acid methyl ester or fatty acid ethyl ester and glycerine, which are recovered separately, in one or several conversion stages, where each conversion stage has a mixing zone and a separator for separating a light phase rich in ester and a heavy phase rich in glycerine, where to the mixing zone of the first conversion stage oil or fat as well as methanol or ethanol and alkaline catalyst are supplied and from the phase rich in glycerine, which was recovered in the first conversion stage, glycerine is separated, characterized in that glycerine recovered from the conversion is mixed with alkali hydroxide and an alkaline, water-containing solution is produced, which contains gylcerine and alkali hydroxide in a molar ratio of 1 : 5 to 10 : 1, that water is removed from the alkaline solution, where mono alkali glycerate is formed, and the glycerate-containing solution is introduced into at least one mixing zone, where the entire catalyst requirement of the conversion is covered for 50 to 100 %.

2. The process as claimed in claim 1, characterized in that glycerine recovered from the conversion is mixed with an amount of alkali hydroxide which corresponds to 0.1 to 1.0 wt-% of the amount of the oil or fat to be converted.

3. The process as claimed in claim 1 or 2, characterized in that the glycerate-containing solution, which is introduced into at least one mixing zone, contains not more than 0.5 wt-% water.

4. The process as claimed in claim 1 or any of the preceding claims, characterized in that the entire catalyst requirement of the conversion of the oil or fat is covered for at least 80 % by the glycerate-containing solution.

## Revendications

1. Procédé de réaction d'huile ou de matière grasse sur le méthanol ou sur l'éthanol et sur un catalyseur alcalin en phase liquide pour obtenir comme produits de l'ester méthylique d'acide gras ou de l'ester éthylique d'acide gras et de la glycérine, qui sont recueillis séparément, en un étage de réaction ou en plusieurs étages de réaction, chaque étage de réaction comportant une zone de mélange et un séparateur destiné à séparer une phase liquide riche en ester et une phase lourde riche en glycérine, de l'huile et de la matière grasse ainsi que du méthanol ou de l'éthanol et un catalyseur alcalin étant envoyés à la zone de mélange du premier étage de réaction et de la glycérine étant séparée de la phase riche en glycérine recueillie dans le premier étage de réaction, caractérisé en ce que l'on mélange la glycérine obtenue par la réaction à de l'hydroxyde de métal alcalin et on produit une solution alcaline aqueuse qui renferme de la glycérine et de l'hydroxyde de métal alcalin en un rapport molaire de 1 : 5 à 10 : 1, en ce qu'on élimine de l'eau de la solution alcaline avec formation de glycérate de métal mono-alcalin et on envoie la solution contenant du glycérate dans au moins une zone de mélange, les besoins totaux en catalyseur pour la réaction étant couverts pour 50 à 100%.

2. Procédé suivant la revendication 1, caractérisé en ce que l'on mélange la glycérine obtenue par la réaction à une quantité d'hydroxyde de métal alcalin qui représente de 0,1 à 1,0 % en poids de la quantité de l'huile ou de la matière grasse qui réagit.

3. Procédé suivant la revendication 1 ou 2, caractérisé en ce que la solution contenant du glycérate que l'on envoie dans au moins une zone de mélange renferme au plus 0,5 % en poids d'eau.

4. Procédé suivant la revendication 1 ou l'une des suivantes, caractérisé en ce que tous les besoins en catalyseur de la réaction de l'huile ou de la matière grasse sont couverts pour au moins 80% par la solution renfermant du glycérate.
